# EUROPÄISCHE PATENTSCHRIFT

(11) **EP 1 309 610 B1**
(45) Veröffentlichungstag und Bekanntmachung des Hinweises auf die Patenterteilung: **31.12.2008**
(21) Anmeldenummer: 01960611.0
(22) Anmeldetag: 02.08.2001
(51) Int. Cl.: C07K 7/00

(54) **PEPTID- UND PEPTIDMIMETIKA-DERIVATE MIT INTEGRIN-INHIBITOR-EIGENSCHAFTEN II**
PEPTIDE AND PEPTIDE MIMETIC DERIVATIVES WITH INTEGRIN INHIBITOR PROPERTIES II
DERIVES PEPTIDIQUES ET DERIVES DE MIMETIQUES PEPTIDIQUES AYANT DES PROPRIETES II D'INHIBITION DE L'INTEGRINE

(30) Priorität: 17.08.2000 DE 10040103
(43) Veröffentlichungstag der Anmeldung: 14.05.2003
(73) Patentinhaber: Biomet Deutschland GmbH, 14167 Berlin (DE)
(72) Erfinder: MEYER, Jörg, 63150 Heusenstamm (DE); NIES, Berthold, 64407 Fränkisch-Crumbach (DE); DARD, Michel, 64342 Seeheim-Jugenheim (DE); HÖLZEMANN, Günter, 64342 Seeheim-Jugenheim (DE); KESSLER, Horst, 65842 Schwalbach (DE); KANTLEHNER, Martin, 85354 Freising (DE); HERSEL, Ulrich, 97250 Erlabrunn (DE); GIBSON, Christoph, 79241 Ihringen (DE); SULYOK, Gabor, 80807 München (DE)
(74) Vertreter: Gross, Felix
(86) Internationale Anmeldenummer: PCT/EP2001/008931
(87) Internationale Veröffentlichungsnummer: WO 2002/014350

(56) Entgegenhaltungen:
- WO-A-92/12727
- DE-A- 19 755 800
- DE-A- 19 755 801

## Beschreibung

Die Erfindung betrifft Verbindungen der Formel I

B-Q-X₁ I

worin
- B: ein bioaktives, zelladhäsionsvermitteindes Molekül, ausgewählt aus der Gruppe 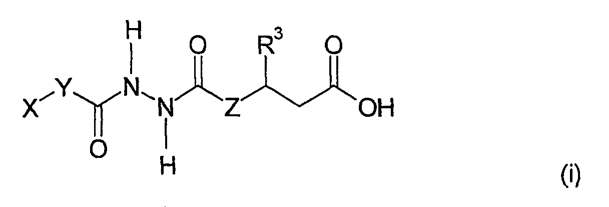
und

Thr-Trp-Tyr-Lys-Ile-Ala-Phe-Gln-Arg-Asn-Arg-Lys (ii)

Trp-Tyr-Lys-Ile-Ala-Phe-Gln-Arg-Asn-Arg-Lys (iii)

Tyr-Lys-Ile-Ala-Phe-Gln-Arg-Asn-Arg-Lys (iv)

Thr-Trp-Tyr-Lys-Ile-Ala-Phe-Gln-Arg-Asn-Arg (v)

Thr-Trp-Tyr-Lys-Ile-Ala-Phe-Gln-Arg-Asn (vi)

Thr-Trp-Tyr-Lys-Ile-Ala-Phe-Gln-Arg (vii),

wobei für **(i)**
- X: H₂N-C(=NH)-NH, Het-NH-, H₂N-C(=NH)-, A-C(=NH)-NH- oder Het-
- Y: -(CH₂)ₙ- oder 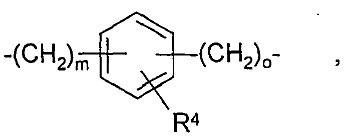 -(CH₂)ₛ-CH(R⁴)-(CH₂)ₜ- oder -(CH₂)ₚ-Het²-(CH₂)_{q}-,
- Z: N-R² oder CH-R²
- R²: H oder Alkyl mit 1 bis 4 C-Atomen
- R³: H, Ar, Het oder A
- R⁴: H, A, Ar, OH,OA, OAr, Arylalkyl, Hal, CN, NO₂, CF₃ oder OCF₃,
- A: COOH, NH₂ oder Alkyl mit 1-6 C-Atomen, unsubstituiert oder substituiert mit COOH oder NH₂
- Ar: unsubstituiertes oder ein-, zwei-oder dreifach durch A, OH, NH₂, OA, CF₃, OCF₃, CN, NO₂ oder Hal substituiertes Phenyl, welches durch ein ein-, zwei oder dreifach durch A, OH, OA, OCF₃, CN, NO₂ oder Hal substituiertes Phenyl in der Art substituiert sein kann, dass ein unsubstituiertes oder substituiertes Biphenyl entsteht,
- Hal: F, Cl, Br oder I,
- Het: einen gesättigten, teilweise oder vollständig ungesättigten mono- oder bicyclischen heterocyclischen Rest mit 5 bis 10 Ringgliedern, wobei 1 bis 3 N-und/oder ein S- oder O-Atome vorliegen können und der heterocyclische Rest ein- oder zweifach durch CN, Hal, OH, NH₂, COOH, OA, CF₃, A, NO₂, Ar oder OCF₃ substituiert sein kann,
- Het²: einen 5- oder 6-gliedrigen aromatischen Heterocyclus mit 1 bis 4 N- und/oder S-Atomen, der unsubstituiert oder ein- oder zweifach durch F, Cl, Br, A, OA oder OCF₃ substituiert sein kann,
- n: 4, 5 oder 6,
- m, o, p, q: 0, 1 oder 2
- s, t: 0, 1, 2, 3, 4, 5
bedeuten;
- Q: fehlt oder ein organisches Spacer-Molekül und
- X ₁: ein Ankermolekül, ausgewählt aus der Gruppe

-CO-CH=CH₂ (viii)

-CO-(CH₂)₁₋₂₀-CO-CH=CH₂ (ix)

-CO-(CH₂)₁₋₂₀-SH (x)

-CO-CH(NH₂)-CH₂-SH (xi)

-NH-(CH₂)₁₋₂₀-CO-CH=CH₂ (xii)

-NH-(CH₂)₂₋₂₀-SH (xiii)

-NH-CH(CO₂H)-CH₂-SH (xiv)

bedeutet,
wobei im Falle der Verbindungen (viii) bis (xi) eine freie Aminogruppe der Gruppe B mit einer freien Carboxylgruppe des Spacer-Moleküls Q oder des Ankermoleküls X bzw. eine freie Aminogruppe des Restes Q mit einer freien Carboxylgruppe des Restes X₁ peptidartig miteinander verknüpft ist, und im Falle der Verbindungen (xii) bis (xiv) eine freie Carboxylgruppe der Gruppe B mit einer freien Aminogruppe des Spacer-Moleküls Q oder des Ankermoleküls X₁ bzw. eine freie Carboxylgruppe des Restes Q mit einer freien Aminogruppe des Restes X ₁ peptidartig miteinander verknüpft ist, sowie deren Salze.

Ähnliche Verbindungen sind aus DE 19932796, DE 19755800 und DE 19831710 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Vor allem wirken sie als Integrin-Inhibitoren, wobei sie insbesondere die Wechselwirkungen der αv-, β₃- oder β₅-Integrin-Rezeptoren mit Liganden hemmen, wie z. B. die Bindung von Fibrinogen an den β₃-Integrinrezeptor. Besondere Wirksamkeit zeigen die Verbindungen im Fall der Integrine αvβ₃, αvβ₅, α_{IIb}β₃ sowie αvβ₁, αvβ₆ und αvβ₈.

Diese Wirkung kann z.B. nach der Methode nachgewiesen werden, die von J.W. Smith et al. in J. Biol. Chem. 265, 12267-12271 (1990) beschrieben wird.
Die Abhängigkeit der Entstehung von Angiogenese von der Wechselwirkung zwischen vaskulären Integrinen und extrazellulären Matrixproteinen ist von P.C. Brooks, R.A. Clark und D.A. Cheresh in Science 264, 569-71 (1994) beschrieben.

Verbindungen der Formel 1, die die Wechselwirkung von Integrinrezeptoren und Liganden, wie z. B. von Fibrinogen an den Fibrinogenrezeptor (Glycoprotein IIb/IIIa) blockieren, verhindern als GPIIb/IIIa-Antagonisten die Ausbreitung von Tumorzellen durch Metastase. Dies wird durch folgende Beobachtungen belegt:
Die Verbreitung von Tumorzellen von einem lokalen Tumor in das vaskuläre System erfolgt durch die Bildung von Mikroaggregaten (Mikrothromben) durch Wechselwirkung der Tumorzellen mit Blutplättchen. Die Tumorzellen sind durch den Schutz im Mikroaggregat abgeschirmt und werden von den Zellen des Immunsystems nicht erkannt.
Die Mikroaggregate können sich an Gefäßwandungen festsetzen, wodurch ein weiteres Eindringen von Tumorzellen in das Gewebe erleichtert wird. Da die Bildung der Mikrothromben durch Fibrinogenbindung an die Fibrinogenrezeptoren auf aktivierten Blutplättchen vermittelt wird, können die GPIIa/IIIb-Antagonisten als wirksame Metastase-Hemmer angesehen werden.

Der (Meth-)acrylrest dient dazu, die Peptide und Peptidmimetika kovalent oder adsorptiv an biokompatible Oberflächen von z.B. Implantaten zu binden, die freie Acrylat- oder Methacrylatreste aufweisen, wie z.B. Polymethylmethacryl-Formkörper (Knochenzemente) oder acrylat- bzw. methacrylathaltige Schichten z.B. auf Metalloberflächen.
Entsprechend dient der Thiolrest der Peptidanbindung z.B. an Goldoberflächen oder an freie Aminogruppen-haltige Oberflächen wie z.B. Kollagen (z.B. mittels Maleinimid), mit Plasma-behandelte Polymer- oder Metalloberflächen (siehe D.M. Ferris, G.D. Moodie, P.M. Dimond, C.W.D. Giovanni, M.G. Ehrlich, R.F. Valentini, Biomaterials 20, 2323-2331(1999); D.F. Meyers et al., J. Immunol. Methods 121,129-142 (1989)) oder mit CVD-Technik behandelte Oberflächen (siehe Lahann, J. et al., Macromol. Rapid Commun. 19, 441-444 (1998))

Gegenstand der Erfindung sind daher die Verbindungen der Formel I zue kovalenten oder adsorptiven Bindung über die funktionelle Gruppe des Restes X₁ an biokompatible Oberflächen.

Es wird in diesem Zusammenhang auf die am gleichen Tag von der Anmelderin eingereichte zweite Anmeldung verwiesen, in der der Rest X₁ eine andere Bedeutung hat.

Die erfindungsgemäßen Peptide und Peptidmimetika ermöglichen nun die Biofunktionalisierung von Biomaterialien, insbesondere Implantaten für alle denkbaren Organe durch deren Beschichtung, wobei vorwiegend die Adhäsion derjenigen Zellspezies stimuliert wird, die jeweils die Gewebeintegration des entsprechenden Biomaterials vollführen sollen. Mit der Verwendung solcher Beschichtungen ist eine beschleunigte und die verstärkte Integration verschiedener Biomaterialien/Implantate mit verbesserter Langzeitstabilität nach deren Einbringen in den Körper zu erzielen.

Die erfindungsgemäßen Peptide binden selektiv an Integrine. Nach Immobilisierung an biokompatiblen Oberflächen, z.B. Implantaten, stimulieren sie die Adhäsion von Zellen, die Integrine tragen.
Nach Beschichtung der Verbindungen auf den Oberflächen, können selektiv diejenigen Zell-Spezies zur Bindung stimuliert werden, die auch nach Implantation im natürlichen Gewebe die Implantatintegration vollführen sollen. So handelt es sich z.B. bei Osteoblasten, Osteoclasten und Endothelzellen um αv-tragende Zellspezies.

Gegenstand der Erfindung sind daher die Verbindungen der Formel I als Integrininhibitoren zur selektiven Zellanreicherung an Implantaten.

Die Verbindungen der Formel I können nach Verankerung an einer biokompatiblen Oberfläche als Arzneimittelwirkstoffe in der Human- und Veterinärmedizin eingesetzt werden, insbesondere können sie als Integrininhibitoren zur Behandlung von durch Implantate verursachten Erkrankungen, Defekten und Entzündungen wie ungenügender und verzögerter Integration von Biomaterialien und Implantaten, von durch Implantate verursachter Thrombose, von Knochen- und Zahndefekten, sowie von osteolytischen Erkrankungen wie Osteoporose, Thrombose, Herzinfarkt, Arteriosklerose, bei der Wundheilung zur Unterstützung der Heilungsprozesse, sowie zur Beschleunigung und Verstärkung des Integrationsprozesses des Implantats bzw. der biokompatiblen Oberfläche in das Gewebe, eingesetzt werden.

Die Verbindungen der Formel I können als antimikrobiell wirkende Substanzen bei Operationen eingesetzt werden, wo Biomaterialien, Implantate, Katheter oder Herzschrittmacher verwendet werden.
Dabei wirken sie antiseptisch. Die Wirksamkeit der antimikrobiellen Aktivität kann durch das von P.Valentin-Weigund et al., in Infection and Immunity, 2851-2855 (1988) beschriebene Verfahren nachgewiesen werden.

Gegenstand der Erfindung sind somit die Verbindungen der Formel I als Integrininhibitoren zur Behandlung von durch Implantate verursachten Erkrankungen, Defekten, Entzündungen und von osteolytischen Erkrankungen wie Osteoporose, Thrombose, Herzinfarkt und Arteriosklerose, sowie zur Beschleunigung und Verstärkung des Integrationsprozesses des Implantats bzw. der biokompatiblen Oberfläche in das Gewebe.

Gegenstand der Erfindung ist ferner die Verwendung von Verbindungen der Formel I zur Herstellung eines Arzneimittels zur Behandlung von durch Implantate verursachten Erkrankungen, Defekten, Entzündungen und von osteolytischen Erkrankungen wie Osteoporose, Thrombose, Herzinfarkt und Arteriosklerose, sowie zur Beschleunigung und Verstärkung des Integrationsprozesses des Implantats bzw. der biokompatiblen Oberfläche in das Gewebe.

Entsprechende thiolankertragende Peptide können kovalent an vergoldete Träger, wie z.B. Implantate, Affinitätschromatographien oder Mikrotiterplatten gebunden werden.

Gegenstand der Erfindung ist auch die Verwendung von Verbindungen der Formel I zur Beschichtung mittels kovalenter oder adsorptiver Bindung von Implantaten für humane und tierische Organe.

Die vor- und nachstehend aufgeführten Abkürzungen von Aminosäureresten stehen für die Reste folgender Aminosäuren:
- Abu: 4-Aminobuttersäure
- Aha: 6-Aminohexansäure, 6-Aminocapronsäure
- Ala: Alanin
- Asn: Asparagin
- Asp: Asparaginsäure
- Arg: Arginin
- Cys: Cystein
- Dab: 2,4-Diaminobuttersäure
- Dap: 2,3-Diaminopropionsäure
- Gln: Glutamin
- Glp: Pyroglutaminsäure
- Glu: Glutaminsäure
- Gly: Glycin
- His: Histidin
- homo-Phe: homo-Phenylalanin
- Ile: Isoleucin
- Leu: Leucin
- Lys: Lysin
- Met: Methionin
- Nle: Norleucin
- Orn: Ornithin
- Phe: Phenylalanin
- Phg: Phenylglycin
- 4-Hal-Phe: 4-Halogen-phenylalanin
- Pro: Prolin
- Ser: Serin
- Thr: Threonin
- Trp: Tryptophan
- Tyr: Tyrosin
- Val: Valin.

Ferner bedeuten nachstehend:
- Ac: Acetyl
- BOC: tert.-Butoxycarbonyl
- CBZ oder Z: Benzyloxycarbonyl
- DCCl: Dicyclohexylcarbodiimid
- DMF: Dimethylformamid
- EDCl: N-Ethyl-N,N'-(dimethylaminopropyl)-carbodiimid
- Et: Ethyl
- FCA: Fluoresceincarbonsäure
- FITC: Fluoresceinisothiocyanat
- Fmoc: 9-Fluorenylmethoxycarbonyl
- FTH: Fluoresceinthioharnstoff
- HATU: O-(7-Azabenzotriazol-1-yl)-N,N,N',N'-Tetramethyluronium-hexafluorophosphat
- HOBt: 1-Hydroxybenzotriazol
- Me: Methyl
- MBHA: 4-Methyl-benzhydrylamin
- Mtr: 4-Methoxy-2,3,6-trimethylphenyl-sulfonyl
- HONSu: N-Hydroxysuccinimid
- OtBu: tert.-Butylester
- Oct: Octanoyl
- OMe: Methylester
- OEt: Ethylester
- POA: Phenoxyacetyl
- Pbf: Pentamethylbenzofuranyl
- Pmc: 2,2,5,7,8-Pentamethylchroman-6-sulfonyl
- Sal: Salicyloyl
- Su: Succinyl
- TFA: Trifluoressigsäure
- Trt: Trityl (Triphenylmethyl).

Sofern die vorstehend genannten Aminosäuren in mehreren enantiomeren Formen auftreten können, so sind vor- und nachstehend, z. B. als Bestandteil der Verbindungen der Formell, alle diese Formen und auch ihre Gemische (z. B. die DL-Formen) eingeschlossen. Ferner können Aminosäuren bzw. die freie Aminogruppe (xi) oder die freie Carboxylgruppe (xiv) als Bestandteil von Verbindungen der Formell, mit entsprechenden an sich bekannten Schutzgruppen versehen sein.
Vor allem Seitenkettenmodifikationen des Arginins, wie sie z.B. bei den nichtpeptidischen aᵥβ₃-Antagonisten vorgenommen wurden (z.B. durch R.Keenan et al., Abstr. Pap. 211th ACS National Meeting (New Orleans, USA) 1996, MEDI 236), können auch bei den Cyclopeptiden eingesetzt werden, wie z.B. Benzimidazolderivate anstelle der Guanidingruppe.

Gegenstand der Erfindung ist ferner ein Implantat, geeignet für humane und tierische Organe, bestehend aus einer Trägermatrix und einer diese Matrix umhüllenden Schicht eines bioaktiven, zelladhäsionsvermittelnden Moleküls, wobei die umhüllende Schicht aus einer Verbindung der Formell gebildet wird, und wobei zwischen Trägermatrix und dieser Verbindung eine kovalente oder adsorptive Bindung vorliegt. Vorzugsweise besteht die Trägermatrix undl oder deren Oberfläche aus einem Metall bzw. Metalloxid oder einem Polymer. Als Polymere kommen bevorzugt Polymethylmethacrylat, Polyhydroxyethylmethacrylat, Polylactit, Polyglycolsäure oder deren Copolymere in Betracht.

Gegenstand der Erfindung ist ferner ein Verfahren zur Herstellung von Verbindungen der Formell nach Anspruch 1 sowie ihrer Salze, dadurch gekennzeichnet, daß man ein bioaktives Molekül B, welches mit Schutzgruppen versehen sein kann, und ein mit Schutzgruppen versehenes Spacer-Anker-Molekül (Q-X₁) bzw. Anker-Molekül (X₁) peptidisch miteinander verknüpft und anschließend die Schutzgruppen abspaltet, und/oder dass man eine basische oder saure Verbindung der Formel 1 durch Behandeln mit einer Säure oder Base in eines ihrer Salze überführt.

Vor- und nachstehend haben die Reste B, Q und X₁ die bei der Formel I angegebene Bedeutung, sofern nicht ausdrücklich etwas anderes angegeben ist.
Q fehlt oder bedeutet ein organisches Spacer-Molekül. Bevorzugt ist dieses ein [CO-(CH₂)ₓ-NH-]ₘ-, [CO-CH₂-(O-CH₂CH₂)_{y}-NH-]ₘ-, [CO-(CH₂)_{z}-CO-]-, [NH-(CH₂)_{z}-NH-]-, [CO-CH₂-(OCH₂CH₂)_{y}-O-CH₂-CO-]-oder ein [NH-CH₂CH₂-(OCH₂CH₂)_{y}-NH-]-Rest sowie deren Kombinationen, wobei für die Indices m, x, y und z die im Anspruch 2 angegebenen Wertebereiche gelten. Als besonders vorteilhaft haben sich die vorgenannten Verbindungen erwiesen, die für m Werte zwischen 1 und 8, für x Werte zwischen 1 und 5 und für y und z Werte zwischen 1 und 6 annehmen können.

X₁ bedeutet ein Ankermolekül, vorzugsweise aus der Gruppe -CO-CH=CH₂, -CO-(CH₂)₁₋₂₀-CO-CH=CH₂, -CO-(CH₂)₁₋₂₀-SH, -CO-CH(NH₂)-CH₂-SH, -NH-(CH₂)₁₋₂₀-CO-CH=CH₂, -NH-(CH₂)₂₋₂₀-SH, oder -NH-CH(CO₂H)-CH₂-SH X bedeutet vorzugsweise H₂N-C(=NH)-NH-, Het-NH-, H₂N-C(=NH)-, A-C(=NH)-NH oder ein Het-Rest.
Y bedeutet vorzugsweise -(CH₂)ₙ- oder 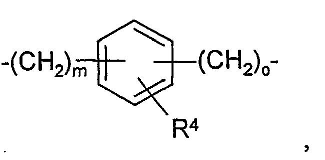 -(CH₂)ₛ-CH(R⁴)-(CH₂)ₜ- oder -(CH₂)ₚ-Het²-(CH₂)_{q}-Rest.
Z bedeutet vorzugsweise N-R² oder CH-R² wobei R² vorzugsweise ein H-Atom oder Alkyl-Rest mit 1 bis 4 C-Atomen sein kann.

R³ bedeutet vorzugsweise ein H-Atom, Ar, Het oder A -Rest, wobei A, Ar und Het eine der zuvor oder nachstehend angegebenen Bedeutungen haben.

R⁴ bedeutet vorzugsweise ein H-Atom, A, Ar, OH, OA, OAr, Arylalkyl, Hal, CN, NO₂, CF₃ oder OCF₃ -Rest. Arylalkyl bedeutet bevorzugt Benzyl, Phenylethyl, Phenylpropyl oder Naphthylmethyl, besonders bevorzugt Benzyl.

A bedeutet vorzugsweise ein COOH-, NH₂- oder Alkyl-Rest mit 1 bis 6, vorzugsweise 1, 2, 3, 4, 5 oder 6 C-Atomen, unsubstituiert oder substituiert mit COOH oder NH₂. A bedeutet vorzugsweise Methyl, weiterhin Ethyl, Propyl, n-Butyl, Isobutyl, sek.-Butyl oder tert. Butyl, ferner auch n-Pentyl, 1-,2- oder 3-Methylbutyl, 1,1-, 1,2- oder 2,2-Dimethylpropyl, 1-Ethylpropyl, Hexyl, 1-,2-,3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl. Besonders bevorzugt für A ist Methyl.

Ar bedeutet vorzugsweise unsubstituiertes oder ein-, zwei- oder dreifach durch A, OH, OA, CF₃, OCF₃, CN, NO₂ oder Hal substituiertes Phenyl, welches durch ein-, zwei- oder dreifach durch A, OH, OA, NH₂, OCF₃, CN, NO₂ oder Hal substituiertes Phenyl in der Art substituiert sein kann, dass ein unsubstituiertes oder substituiertes Biphenyl entsteht.

Ar bedeutet daher bevorzugt Phenyl, o-, m- oder p-Methylphenyl, o-, m-oder p-Ethylphenyl, o-, m- oder p-Propylphenyl, o-, m- oder p-Isopropylphenyl, o-, m- oder p-tert. Butylphenyl, o-, m- oder p-Hydroxyphenyl, o-, m- oder p-Methoxyphenyl, o-, m- oder p- Ethoxyphenyl, o-, m-, p-Trifluormethylphenyl, o-, m-, p-Trifluormethoxyphenyl, o-, m- oder p-Fluorphenyl, o-, m- oder p-Chlorphenyl, o-, m- oder p-Bromphenyl, o-, m-oder p-Nitrophenyl.

Het bedeutet einen gesättigten, teilweise oder vollständig ungesättigten mono- oder bicyclischen heterocyclischen Rest mit 5 bis 10 Ringgliedern, wobei 1 bis 3 N- und/ oder 1S- oder O-Atome vorliegen können und der heterocyclische Rest ein- oder zweifach durch CN, Hal, OH, NH₂, COOH, OA, CF₃, A, NO₂, Ar oder OCF₃ substituiert sein kann.

Het ist vorzugsweise ein o-, m-, p-substituiertes Pyridyl, ein 2-, 4-, 5- oder 6-substituiertes Pyrimidyl oder ein 3-, 4-, 5- oder 6-substituiertes Pyridazyl, das bevorzugt unsubstituiert oder substituiert durch eine Methyl-, Ethyl-, Propylgruppe oder eine Methylamino-, Ethylamino- oder Propylaminogruppe ist [bezieht sich auf alle der drei genannten Heteroaromaten], sowie ein 2- substituiertes Benzimidazolyl, das unsubstituiert oder durch eine 3-Methyl-, 3-Ethyl- oder 3-Benzylgruppe substituiert ist, sowie ein 2-substituiertes Dihydroimidazolyl, Tetrahydropyrimidyl oder Tetrahydropyridyl.
Beispiele, die in Het bevorzugt enthalten sind:

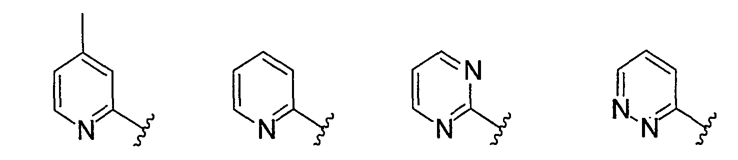

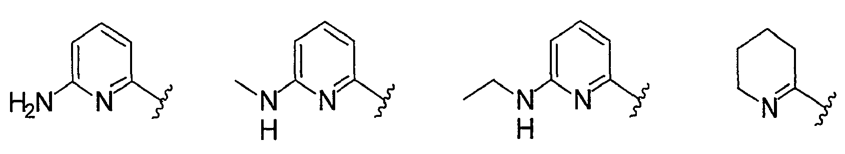

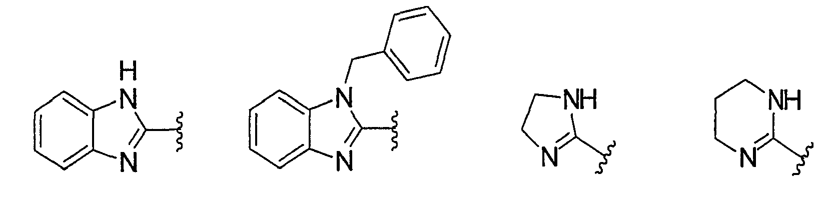

Het¹ bedeutet einen 5- oder 6-gliedrigen aromatischen Heterocyclus mit 1 bis 4 N- und/ oder S-Atomen, der unsubstituiert oder ein- oder zweifach durch F, Cl, Br, A, OA oder OCF₃ substituiert sein kann.

Het¹ ist vorzugsweise ein 2,4-, 3,5-, 2,5-disubstituiertes Pyridyl oder ein 2,4-, 2,5-, 2,6-, 4,6-disubstituiertes Pyrimidyl, ein 2,4-, 2,5-disubstituiertes 1,3-Oxazolyl oder 1,3-Thiazolyl.

OA bedeutet vorzugsweise Methoxy, Ethoxy, Propoxy oder Butoxy, ferner auch Pentyloxy oder Hexyloxy.

Hal bedeutet vorzugsweise F, Cl, oder Br, aber auch I.

Die Indices n, m, o, p, q, s und t haben die im Anspruch 1 angegebene Bedeutung, sofern nicht ausdrücklich etwas anderes angegeben ist.

Die Verbindungen der Formel 1 können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat.

Besonders bevorzugt sind folgende Verbindungen der Formel I:
a) 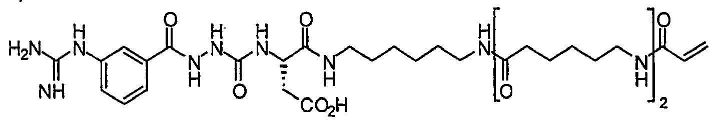
b) Thr-Trp-Tyr-Lys-Ile-Ala-Phe-Gln-Arg-Asn-Arg-Lys-Aca-Aca-Cys
c) Cys-Aca-Aca-Thr-Trp-Tyr-Lys-Ile-Ala-Phe-Gln-Arg-Asn-Arg-Lys
d) Lys-Ile-Ala-Phe-Gln-Arg-Asn-Arg-Lys-Aca-Aca-Cys
e) Cys-Aca-Aca-Thr-Trp-Tyr-Lys-Ile-Ala-Phe-Gln-Arg
f) Phe-Gln-Arg-Asn-Arg-Lys-Aca-Aca-Cys
g) Cys-Aca-Aca-Thr-Trp-Tyr-Lys-Ile-Ala

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart oder The Journal of Biological Chemistry, Vol. 271, No. 44, pp. 27221 ff. (1996)) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls gewünscht, auch in situ gebildet werden, so dass man sie aus dem Reaktionsgemisch nicht isoliert, sondern sofort weiter zu den Verbindungen der Formel I umsetzt.

Die Fragmentkupplung erfolgt in einem inerten Lösungsmittel, wobei ein Carbonsäurefragment (Acrylat- oder Thiollinker) mit HATU, HOAt und 2,4,6-Collidin in DMF gelöst wird und anschließend mit dem Aminfragment (lineares Peptid, Peptidmimetikum) versetzt wird bzw. auch umgekehrt (Linker = Aminfragment; Peptid/Mimetikum = Carbonsäurefragment).

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan, Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, N-Methylpyrrolidon, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat, Wasser oder Gemische der genannten Lösungsmittel.

### Allgemeine Arbeitstechniken:

Die Reagenzien wurden von den Firmen Aldrich, Bachem, Fluka, Neosystem und Novabiochem bezogen und ohne weitere Aufreinigung verwendet. Das Tritylchlorid-Polystyrol-Harz (TCP-Harz) stammte von der Firma PepChem.

(RP9)-HPLC-Analytik und semipräparative Trennungen wurden an Geräten der Firmen Waters und Beckman mit UV-Detektor (Uvicord) von Knauer und Amersham Pharmacia Biotech durchgeführt. Die UV-Detektion erfolgte bei der Wellenlänge 220 nm. Folgende Säulenmaterialien wurden für die Analytik (Durchmesser 4 mm) und semipräparative Trennung (Durchmesser 21 mm bzw. 40 mm) benutzt: Nucleosil RP18 5µ, Nucleosil RP18 HD 5µ und Nucleosil RP 18 7µ von Macherey & Nagel). Als Eluent dienten Laufmittelgemische aus Acetonitril und Wasser mit jeweils 0.1 Vol-% TFA im Gradientenbetrieb.

Die ESI-Massenspektren wurden mit einem Gerät der Fa. Finnigan vom Typ LCQ durchgeführt.

NMR-Spektren wurden an einem Bruker DMX 500 aufgenommen. Interner Standard für chemische Verschiebungen von ¹H und ¹³C war das Lösungsmittel von DMSO-d₅: 2.49 ppm (¹H-NMR) und 39.5 ppm (¹³C-NMR).

Die Festphasensynthesen wurden in 10 ml-Kunststoffspritzen mit PP-Fritten der Firma Vetter-Laborbedarf (Tübingen) durchgeführt. Die Kunststoffspritzen wurden zur Durchmischung der Harzsuspensionen mit ca. 15 rpm über Kopf rotiert.
Die Umsetzungen des aktivierten azaGly und die Guanylierungen an der Festphase wurden unter Ausschluß von Luftfeuchtigkeit durchgeführt.

Das nachfolgende Beispiel beschreibt die erfindungsgemäßen Verbindungen anhand der mehrstufigen Synthese eines aza-RGD-Mimetikums mit Acrylatanker.

### Beispiel 1:

### Synthese von Gua-Mab-azaGly-Asp-Hda-εAhx-εAhx-Aca (4)

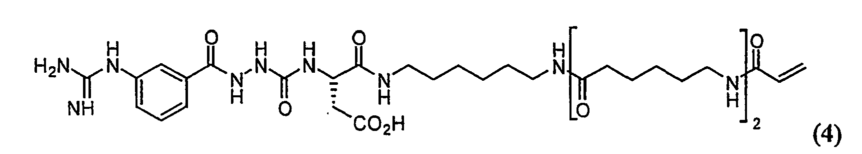

### Gua(Boc)₂-Mab-azaGly-Asp(Bu)-OH (1):

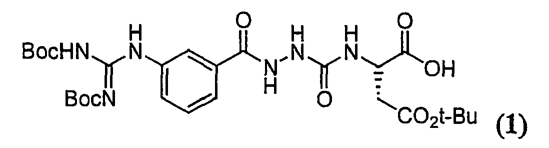

Asp(Bu)-TCP-Harz (1.00 g, 0.6 mmol/g, 0.6 mmol) ergab nach einer mehrstufigen bekannten Synthese (s. Gibson, C.; Goodman, S.L.; Hahn, D. ; Hölzemann, G.; Kessler, H. Novel Solid-Phase Synthesis of Azapeptides and Azapeptoides via Fmoc-Strategy and its Application in the Synthesis of RGD-Mimetics. J. Org. Chem. 1999, 64, 7388-7394 und Gibson, C.; Kessler, H. 2-Fluoropyrimidine as an efficient reagent in solidphase synthesis of N-aryl- and N-alkyl-N-pyrimidin-2-ylamines. Tetrahydron Lett. 2000, 41, 1725-1728) die Verbindung **1** (75.5 mg, 21% Rohausbeute) als farbloses Öl.

HPLC (10-90% in 30 min) Rₜ = 21.9 min; ESI-MS: m/z 1239.1 (30) [2M + Na⁺], 1217.0 (30) [2M + H⁺], 631.1 (30) [M + Na⁺], 609.0 (100) [M + H⁺].

### Gua(Boc)₂-Mab-azaGly-Asp(Bu)-Hda-H (2):

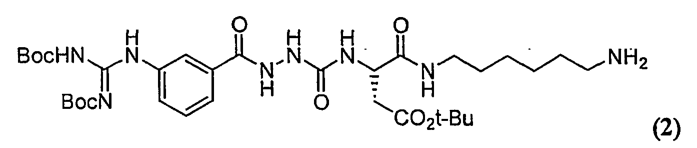

Gua(Boc)₂-Mab-azaGly-Asp(Bu)-OH (**1**) (75.2 mg, 0.123 mmol), HOAt (16.8 mg, 0.124 mmol, 1.0 Äquiv.) und HATU (56.5 mg, 0.148 mmol, 1.2 Äquiv.) wurden in wasserfreiem DMF (1.5 mL) gelöst und anschließend mit Collidin (163 µL, 149 mg, 1.23 mmol, 10 Äquiv.) versetzt. Nach 2 h wurde diese Lösung unter heftigem Rühren bei 10 °C innerhalb 5 min zu einer Lösung von 1,6-Diaminohexan (0.287 g, 2.47 mmol, 20 Äquiv.) in DMF (2 mL) getropft. Man beließ die Reaktion 10 min bei dieser Temperatur und rührte dann weitere 70 min bei Raumtemperatur. Anschließend wurde das Reaktionsgemisch im Ölpumpenvakuum eingeengt. Der Rückstand wurde in CH₂Cl₂ (15 mL) aufgenommen und mit H₂O (2 × 15 mL) extrahiert. Einengen im Vakuum ergab die Verbindung **2** (82.2 mg, 95% Rohausbeute) als braunen Feststoff.

HPLC (10-90% in 30 min) Rₜ = 20.1 min; ESI-MS: *m*/*z* 1435.2 (30) [2M + Na⁺], 1413.2 (30) [2M + H⁺], 729.2 (60) [M + Na⁺], 707.2 (100) [M + H⁺].

### 2:1-Gemisch von Gua(Boc)-Mab-azaGly-Asp(Bu)-Hda-εAhx-εAhx-Aca

### (3a) und Gua(Boc)₂-Mab-azaGly-Asp(Bu)-Hda-εAhx-εAhx-Aca (3b):

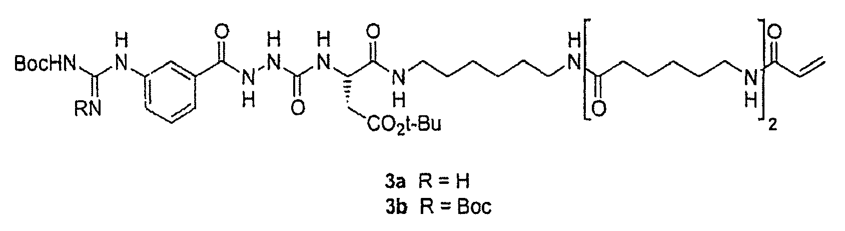

6-{[6-(allanoylamino)hexanoyl]amino}hexansäure (Aca-εAhx-εAhx-OH) (69 mg, 0.23 mmol, 2.0 Äquiv.) und HATU (97 mg, 0.26 mmol, 2.2 Äquiv) wurden in wasserfreiem DMF (2.5 mL) gelöst und anschließend mit Collidin (340 µL, 310 mg, 2.6 mmol, 22 Äquiv.) versetzt. Nach 1 h wurde unter Rühren innerhalb 5 min eine Lösung von Gua(Boc)₂-Mab-azaGly-Asp(Bu)-Hda-H (2) (82.0 mg, 0.116 mmol) in DMF (1 mL) zugetropft. Nach 2 h wurde das Reaktionsgemisch im Ölpumpenvakuum eingeengt. Der Rückstand wurde in einem Lösungsmittelgemisch aus CH₂Cl₂ (1 mL) und TFE (250 µL) aufgenommen und langsam zu CH₃CN (35 mL) getropft. Es bildete sich ein farbloser Niederschlag, der durch Zentrifugieren und Abdekandieren abgetrennt wurde. Entfernen des Lösungmittels im Vakuum und Reinigung per HPLC (30-80% in 30 min) ergab ein Gemisch von **3a** und **3b** im HPLC-Integralverhältnis von 2:1 (33.9 mg, 32%) als farblosen Feststoff.

### 3a:

HPLC (10-90% in 20 min) Rₜ = 11.8 min; ESI-MS: *m*/*z* 909.3 (70) [M + Na⁺], 887.3 (40) [M + H⁺].

### 3b:

HPLC (10-90% in 20 min) Rₜ = 16.8 min; ESI-MS: m/z 1009.3 (90) [M + Na⁺].

### Gua-Mab-azaGly-Asp-Hda-εAhx-εAhx-Aca (4):

Ein 2:1-Gemisch von **3a** und **3b** (33 mg, 36 µmol) wurde in CH₂Cl₂ (0.5 mL) suspendiert und anschließend mit einem 95:5 TFA/H₂O-Gemisch versetzt. Nach 1 h wurde das Reaktionsgemisch im Vakuum eingeengt. Lyophilisieren aus H₂O ergab die Verbindung **4** (29.0 mg, 95%, verunreinigt mit 10% der entguanylierten Verbindung) als farblosen Feststoff.

¹H-NMR (500 MHz, DMSO): δ = 10.31 (s, 1H, Ar-CO-N*H*-NH), 10.02 (s, 1H, NH-Ar), 8.24 (s, 1H, Ar-CO-NH-NH), 8.04 (m_{c}, 1H, CO-NH) 7.77 (d, *J* = 7.8 Hz, 1H, arom), 8.24-7.50 (m, 8H, 2 × Ar-H, *H*₂N-CN*H*_{Z}-NH, 2 × CO-NH), 7.41 (d, *J* = 7.9 Hz, 1H, arom), 7.17-7.09 (m, 1H, CO-NH), 6.80 (br. s, 1H, NH-NH-CO-N*H*), 6.19 (dd, *J* = 17.2, 10.1 Hz, 1H, CO-C*H*=CH₂), 6.04 (dd, *J* = 17.1, 2.1 Hz, 1H, CO-CH=C*H*^{cis}H^{trans}), 5.54 (dd, *J* = 10.1, 2.1 Hz, 1H, CO-CH=CH^{cis}*H*^{trans}), 4.42 (dt, *J* = *J'* = 7.9 Hz, 1H, NH-C*H*-CO), 3.13-2.94 (m, 8H, 4 × CO-NH-C*H*₂) AB-Signal (δ_{A} = 2.63, δ_{B} = 2.55, *J*_{AB} = 16.1, zusätzlich aufgespalten durch *J*_{A,H(α)} = 5.4 Hz und *J*_{B,H(α)} = 7.2 Hz, 2H, CH-C*H*₂-CO₂H), 2.01 (t, *J* = 7.3 Hz, 4H, 2 × CO-CH₂), 1.51-1.15 (m, 20H, aliphat.); ¹³C-NMR (125.0 MHz, DMSO): δ = 172.0, 171.83, 171.76, 170.6. 164.4, 157.6, 155.8, 135.6, 133.9, 131.9, 129.8, 127.6, 125.3, 124.7, 123.4, 50.1, 38.7, 38.4, 38.3, 38.2, 35.3, 29.0, 28.9, 28.8, 26.1, 25.9, 25.0; HPLC (10-80% in 30 min) Rₜ = 10.6 min; ESI-MS: *m*/*z* 753.3 (10) [M + Na⁺], 731.3 (100) [M + H⁺].

### Beispiel für Zelladhäsionstest

Es wurde die Adhäsion von Maus-MC3T3 H1-Osteoblastenkulturen in vitro an Peptid-beschichteten Materialoberflächen untersucht. Dabei wurden 50.000 Zellen/cm² angesät und nach einstündiger Inkubation in serumfreiem Medium bei 37°C / 95 % Luftfeuchte der Anteil adherierter Zellen bestimmt.

### Zellanhaftungsrate [%] = adherierte Zellen / angesäte Zellen x 100

Peptid: Zellanhaftungsrate [%]
Gua-Mab-azaGly-Asp-Hda-εAhx-εAhx-Aca: 75
Thr-Trp-Tyr-Lys-Ile-Ala-Phe-Gln-Arg-Asn-Arg-Lys-Aca-Aca-Cys: 105
Cys-Aca-Aca-Thr-Trp-Tyr-Lys-Ile-Ala-Phe-Gln-Arg-Asn-Arg-Lys: 101
Lys-Ile-Ala-Phe-Gln-Arg-Asn-Arg-Lys-Aca-Aca-Cys: 96
Cys-Aca-Aca-Thr-Trp-Tyr-Lys-Ile-Ala-Phe-Gln-Arg: 17
Phe-Gln-Arg-Asn-Arg-Lys-Aca-Aca-Cys: 13
Cys-Aca-Aca-Thr-Trp-Tyr-Lys-Ile-Ala: 7

Die Beschichtung von mit Rinderserumalbumin (BSA) vorbeschichteten Kulturoberflächen aus Polystyrol mit Thiolpeptiden ist aus dem Stand der Technik bekannt (siehe DE 198 18098 (Merck Patent GmbH), Bsp. 2). Auch die Beschichtung von PMMA-Oberflächen mit Acrylatpeptiden ist in der DE 198 18098, Bsp. 3 beschrieben.

## Patentansprüche

1. Verbindung der Formel 1
B-Q-X₁ 1
worin
B ein bioaktives, zelladhäsionsvermittelndes Molekül, ausgewählt aus der Gruppe 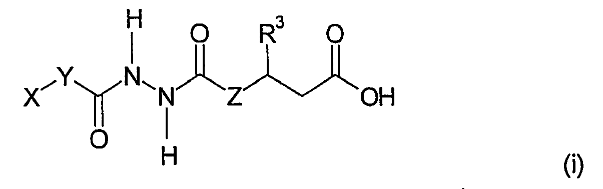 und
Thr-Trp-Tyr-Lys-Ile-Ala-Phe-Gln-Arg-Asn-Arg-Lys (ii)
Trp-Tyr-Lys-Ile-Ala-Phe-Gln-Arg-Asn-Arg-Lys (iii)
Tyr-Lys-Ile-Ala-Phe-Gln-Arg-Asn-Arg-Lys (iv)
Thr-Trp-Tyr-Lys-Ile-Ala-Phe-Gln-Arg-Asn-Arg (v)
Thr-Trp-Tyr-Lys-Ile-Ala-Phe-Gln-Arg-Asn (vi)
Thr-Trp-Tyr-Lys-Ile-Ala-Phe-Gln-Arg (vii),
wobei für **(i)**
X H₂N-C(=NH)-NH, Het-NH-, H₂N-C(=NH)-, A-C(=NH)-NH- oder Het-
Y -(CH₂)ₙ- oder 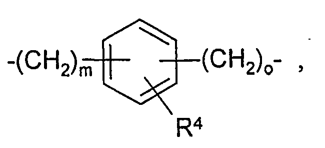 -(CH₂)ₛ-CH(R⁴)-(CH₂)ₜ- oder -(CH₂)ₚ-Het¹-(CH₂)_{q}-,
Z N-R² oder CH-R²
R² H oder Alkyl mit 1 bis 4 C-Atomen
R³ H, Ar, Het oder A
R⁴ H, A, Ar, OH,OA, OAr, Arylalkyl, Hal ,CN, NO₂, CF₃ oder OCF₃,
A COOH, NH₂ oder Alkyl mit 1-6 C-Atomen, unsubstituiert oder substituiert mit COOH oder NH₂
Ar unsubstituiertes oder ein-, zwei-oder dreifach durch A, OH, NH₂, OA, CF₃, OCF₃, CN, NO₂ oder Hal substituiertes Phenyl, welches durch ein ein-, zwei oder dreifach durch A, OH, OA, OCF₃, CN, NO₂ oder Hal substituiertes Phenyl in der Art substituiert sein kann, dass ein unsubstituiertes oder substituiertes Biphenyl entsteht,
Hal F, Cl, Br oder I,
Het einen gesättigten, teilweise oder vollständig ungesättigten mono- oder bicyclischen heterocyclischen Rest mit 5 bis 10 Ringgliedern, wobei 1 bis 3 N-und/oder ein S- oder O-Atome vorliegen können und der heterocyclische Rest ein- oder zweifach durch CN, Hal, OH, NH₂, COOH, OA, CF₃, A, NO₂, Ar oder OCF₃ substituiert sein kann,
Het¹ einen 5- oder 6-gliedrigen aromatischen Heterocyclus mit 1 bis 4 N- und/oder S-Atomen, der unsubstituiert oder ein- oder zweifach durch F, Cl, Br, A, OA oder OCF₃ substituiert sein kann,
n 4, 5 oder 6,
m, o, p, q 0, 1 oder 2
s, t 0, 1, 2, 3, 4, 5
bedeuten;
Q fehlt oder ein organisches Spacer-Molekül und
X₁ ein Ankermolekül, ausgewählt aus der Gruppe
-CO-CH=CH₂ (viii)
-CO-(CH₂)₁₋₂₀-CO-CH=CH₂ (ix)
-CO-(CH₂)₁₋₂₀-SH (x)
-CO-CH(NH₂)-CH₂-SH (xi)
-NH-(CH₂)₁₋₂₀-CO-CH=CH₂ (xii)
-NH-(CH₂)₂₋₂₀-SH (xiii)
-NH-CH(CO₂H)-CH₂-SH (xiv)
bedeutet,
wobei im Falle der Verbindungen (viii) bis (xi) eine freie Aminogruppe der Gruppe B mit einer freien Carboxylgruppe des Spacer-Moleküls Q oder des Ankermoleküls X₁ bzw. eine freie Aminogruppe des Restes Q mit einer freien Carboxylgruppe des Restes X₁ peptidartig miteinander verknüpft ist, und im Falle der Verbindungen (xii) bis (xiv) eine freie Carboxylgruppe der Gruppe B mit einer freien Aminogruppe des Spacer-Moleküls Q oder des Ankermoleküls X₁ bzw. eine freie Carboxylgruppe des Restes Q mit einer freien Aminogruppe des Restes X ₁ peptidartig miteinander verknüpft ist,
sowie deren Salze.

2. Verbindung nach Anspruch 1 , worin Q ausgewählt ist aus der Gruppe
[CO-(CH₂)ₓ-NH-]ₘ, (xvii)
[CO-CH₂-(O-CH₂CH₂)_{y} -NH-]ₘ, (xviii)
[CO-(CH₂)_{z}-CO-] (xix)
[NH-(CH₂)_{z}-NH-] (xx)
[CO-CH₂-(OCH₂CH₂)_{y}-O-CH₂-CO-] (xxi)
[NH-CH₂CH₂-(OCH₂CH₂)_{y}-NH-] (xxii)
sowie deren Kombination
worin
m jeweils unabhängig voneinander 1 bis 20
x 1 bis 12
y 1 bis 50
z 1 bis 12 ist.

3. Verbindung nach Anspruch 1 , worin Q ausgewählt ist aus der Gruppe
[CO-(CH₂)ₓ-NH-]ₘ, (xvii)
[CO-CH₂-(O-CH₂CH₂)_{y}-NH-]ₘ, (xviii)
[CO-(CH₂)_{z}-CO-] (xix)
[NH-(CH₂)_{z}-NH-] (xx)
[CO-CH₂-(OCH₂CH₂)_{y}-O-CH₂-CO-] (xxi)
[NH-CH₂CH₂-(OCH₂CH₂)_{y}-NH-] (xxii)
sowie deren Kombination
worin
m jeweils unabhängig voneinander 1 bis 8
x 1 bis 5
y 1 bis 6 und
z 1 bis 6 ist.

4. Verbindungen der Formel 1 gemäß Anspruch 1
a) 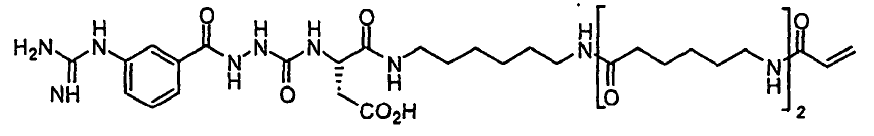
b) Thr-Trp-Tyr-Lys-Ile-Ala-Phe-Gln-Arg-Asn-Arg-Lys-Aca-Aca-Cys
c) Cys-Aca-Aca-Thr-Trp-Tyr-Lys-Ile-Ala-Phe-Gln-Arg-Asn-Arg-Lys
e) Cys-Aca-Aca-Thr-Trp-Tyr-Lys-Ile-Ala-Phe-Gln-Arg.

5. Verbindungen der Formel 1 gemäß Anspruch 1, mit B ausgewählt aus (ii), (iv) oder (v):
d) Lys-Ile-Ala-Phe-Gln-Arg-Asn-Arg-Lys-Aca-Aca-Cys
f) Phe-Gln-Arg-Asn-Arg-Lys-Aca-Aca-Cys
g) Cys-Aca-Aca-Thr-Trp-Tyr-Lys-Ile-Ala

6. Verbindung nach einem der Ansprüche 1 bis 5 als Arzneimittel zur Behandlung von durch Implantate verursachten Erkrankungen, Defekten, Entzündungen und von osteolytischen Erkrankungen wie Osteoporose, Thrombose, Herzinfarkt und Arteriosklerose, sowie zur Beschleunigung und Verstärkung des Integrationsprozesses des Implantats bzw. der biokompatiblen Oberfläche in das Gewebe.

7. Implantat, geeignet für humane und tierische Organe, bestehend im wesentlichen aus einer Trägermatrix und einer diese Matrix umhüllenden Schicht eines bioaktiven, zelladhäsionsvermittelnden Moleküls, **dadurch gekennzeichnet, dass** die umhüllende Schicht aus einer Verbindung gemäß der Ansprüche 1 bis 5 gebildet wird, wobei zwischen Trägermatrix und dieser Verbindung eine kovalente oder adsorptive Bindung vorliegt.

8. Implantat nach Anspruch 7, **dadurch gekennzeichnet, dass** die Trägermatrix und/oder deren Oberfläche ein Metall oder ein Metalloxid ist.

9. Implantat nach Anspruch 7, **dadurch gekennzeichnet, dass** die Trägermatrix und/oder deren Oberfläche ein Polymer ist.

10. Implantat nach Anspruch 9, **dadurch gekennzeichnet, dass** das Polymer Polymethylmethacrylat, Polyhydroxyethylmethacrylat oder deren Copolymere ist.

11. Verfahren zur Herstellung einer Verbindung der Formel 1 nach Anspruch 1 sowie ihrer Salze, **dadurch gekennzeichnet, dass** man ein bioaktives Molekül B, welches mit Schutzgruppen versehen sein kann, und ein mit Schutzgruppen versehenes Spacer-Anker-Molekül (Q-X₁) bzw. Ankermolekül (X₁) peptidisch miteinander verknüpft und anschließend die Schutzgruppen abspaltet, und/oder dass man eine basische oder saure Verbindung der Formel 1 durch Behandeln mit einer Säure oder Base in eines ihrer Salze überführt.

12. Verwendung einer Verbindung nach einem der Ansprüche 1 bis 5 zur Herstellung eines Arzneimittels zur Behandlung von durch Implantate verursachten Erkrankungen, Defekten, Entzündungen und von osteolytischen Erkrankungen wie Osteoporose, Thrombose, Herzinfarkt und Arteriosklerose, sowie zur Beschleunigung und Verstärkung des Integrationsprozesses des Implantats bzw. der biokompatiblen Oberfläche in das Gewebe.

13. Verwendung einer Verbindung nach einem der Ansprüche 1 bis 5 zur Beschichtung mittels kovalenter oder adsorptiver Bindung von Implantaten für humane und tierische Organe.

## Claims

1. Compound of the formula 1
B-Q-X₁ 1
in which
B is a bioactive, cell adhesion-mediating molecule, selected from the group 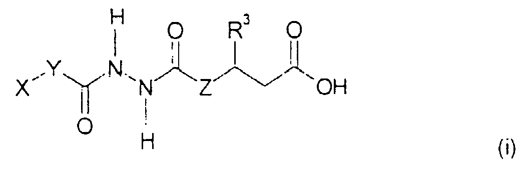 and
Thr-Trp-Tyr-Lys-Ile-Ala-Phe-Gln-Arg-Asn-Arg-Lys (ii)
Trp-Tyr-Lys-Ile-Ala-Phe-Gln-Arg-Asn-Arg-Lys (iii)
Tyr-Lys-Ile-Ala-Phe-Gln-Arg-Asn-Arg-Lys (iv)
Thr-Trp-Tyr-Lys-Ile-Ala-Phe-Gln-Arg-Asn-Arg (v)
Thr-Trp-Tyr-Lys-Ile-Ala-Phe-Gln-Arg-Asn (vi)
Thr-Trp-Tyr-Lys-Ile-Ala-Phe-Gln-Arg (vii),
where for **(i)**
X is H₂N-C (=NH) -NH, Het-NH-, H₂N-C(=NH)-, A-C(=NH)-NH- or Het-,
Y is -(CH₂)ₙ- or 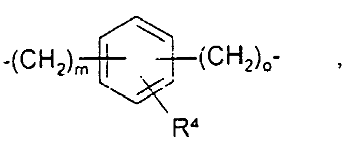 -(CH₂)ₛ-CH(R⁴)-(CH₂)ₜ- or -(CH₂)ₚ-Het¹-(CH₂)_{q}-,
Z is N-R² or CH-R²,
R² is H or alkyl having 1 to 4 C atoms,
R³ is H, Ar, Het or A,
R⁴ is H, A, Ar, OH, OA, OAr, arylalkyl, Hal, CN, NO₂, CF₃ or OCF₃,
A is COOH, NH₂ or alkyl having 1-6 C atoms, which is unsubstituted or substituted by COOH or NH₂,
Ar is phenyl which is unsubstituted or mono-, di- or trisubstituted by A, OH, NH₂, OA, CF₃, OCF₃, CN, NO₂ or Hal, which can be substituted by a phenyl which is mono-, di- or trisubstituted by A, OH, OA, OCF₃, CN, NO₂ or Hal in such a way that an unsubstituted or substituted biphenyl results,
Hal is F, Cl, Br or I,
Het is a saturated, partly or completely unsaturated mono- or bicyclic heterocyclic radical having 5 to 10 ring members, where 1 to 3 N and/or an S or O atom(s) can be present and the heterocyclic radical can be mono- or disubstituted by CN, Hal, OH, NH₂, COOH, OA, CF₃, A, NO₂, Ar or OCF₃,
Het¹ is a 5- or 6-membered aromatic heterocycle having 1 to 4 N and/or S atoms, which can be unsubstituted or mono- or disubstituted by F, Cl, Br, A, OA or OCF₃,
n is 4, 5 or 6,
m, o, p, q are 0, 1 or 2,
s, t are 0, 1, 2, 3, 4, 5;
Q is absent or is an organic spacer molecule and
X₁ is an anchor molecule, selected from the group
-CO-CH=CH₂ (viii)
-CO-(CH₂)₁₋₂₀-CO-CH=CH₂ (ix)
-CO-(CH₂)₁₋₂₀-SH (x)
-CO-CH(NH₂)-CH₂-SH (xi)
-NH-(CH₂)₁₋₂₀-CO-CH=CH₂ (xii)
-NH-(CH₂)₂₋₂₀-SH (xiii)
-NH-CH(CO₂H)-CH₂-SH (xiv),
where in the case of the compounds (viii) to (xi) a free amino group of the group B with a free carboxyl group of the spacer molecule Q or of the anchor molecule X₁ or a free amino group of the radical Q with a free carboxyl group of the radical X₁ is linked to one another like peptides, and in the case of the compounds (xii) to (xiv) a free carboxyl group of the group B with a free amino group of the spacer molecule Q or of the anchor molecule X₁ or a free carboxyl group of the radical Q with a free amino group of the radical X₁ are linked to one another like peptides, and their salts.

2. Compound according to Claim 1, in which Q is selected from the group
[CO-(CH₂₎ₓ-NH-]ₘ, (xvii)
[CO-CH₂-(O-CH₂CH₂)_{y}-NH-]ₘ, (xviii)
[CO-(CH₂)_{z}-CO-] (xix)
[NH-(CH₂)_{z}-NH-] (xx)
[CO-CH₂-(OCH₂CH₂)_{y}-O-CH₂-CO-] (xxi)
[NH-CH₂CH₂-(OCH₂CH₂)_{y}-NH-] (xxii)
and their combination
in which
m in each case independently of one another is 1 to 20,
x is 1 to 12,
y is 1 to 50
z is 1 to 12.

3. Compound according to Claim 1, in which Q is selected from the group
CO-CH₂)ₓ-NH-]ₘ, (xvii)
[CO-CH₂-(O-CH₂CH₂)_{y} -NH-]ₘ, (xviii)
[CO-(CH₂)_{z}-CO-] (xix)
[NH-(CH₂)_{z}-NH-] (xx)
[CO-CH₂-(OCH₂CH₂)_{y}-O-CH₂-CO-] (xxi)
[NH-CH₂CH₂-(OCH₂CH₂)_{y}-NH-] (xxii)
and their combination
in which
m in each case independently of one another is 1 to 8,
x is 1 to 5,
y is 1 to 6 and
z is 1 to 6.

4. Compounds of the formula 1 according to Claim 1:
a) 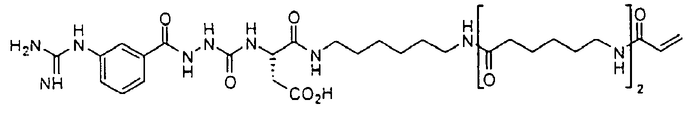
b) Thr-Trp-Tyr-Lys-Ile-Ala-Phe-Gln-Arg-Asn-Arg-Lys-Aca-Aca-Cys
c) Cys-Aca-Aca-Thr-Trp-Tyr-Lys-Ile-Ala-Phe-Gln-Arg-Asn-Arg-Lys
e) Cys-Aca-Aca-Thr-Trp-Tyr-Lys-Ile-Ala-Phe-Gln-Arg

5. Compounds of the formula 1 according to Claim 1, with B selected from (ii), (iv) or (v):
d) Lys-Ile-Ala-Phe-Gln-Arg-Asn-Arg-Lys-Aca-Aca-Cys
f) Phe-Gln-Arg-Asn-Arg-Lys-Aca-Aca-Cys
g) Cys-Aca-Aca-Thr-Trp-Tyr-Lys-Ile-Ala

6. Compound according to one of Claims 1 to 5 as a medicament for the treatment of disorders, defects and inflammations caused by implants, and of osteolytic disorders such as osteoporosis, thrombosis, cardiac infarct and arteriosclerosis, and also for the acceleration and strengthening of the integration process of the implant or of the biocompatible surface into the tissue.

7. Implant, suitable for human and animal organs, consisting essentially of a carrier matrix and a layer of a bioactive, cell adhesion-mediating molecule surrounding this matrix, **characterized in that** the surrounding layer is formed from a compound according to Claims 1 to 5, a covalent or adsorptive bond being present between carrier matrix and this compound.

8. Implant according to Claim 7, **characterized in that** the carrier matrix and/or its surface is a metal or a metal oxide.

9. Implant according to Claim 7, **characterized in that** the carrier matrix and/or its surface is a polymer.

10. Implant according to Claim 9, **characterized in that** the polymer is polymethyl methacrylate, polyhydroxyethyl methacrylate or copolymers thereof.

11. Process for the preparation of a compound of the formula 1 according to Claim 1, and of its salts, **characterized in that** a bioactive molecule B which can be provided with protective groups, and a spacer-anchor molecule (Q-X₁) or anchor molecule (X₁) provided with protective groups are linked to one another in peptide fashion and the protective groups are then removed, and/or **in that** a basic or acidic compound of the formula 1 is converted into one of its salts by treating with an acid or base.

12. Use of a compound according to one of Claims 1 to 5 for the production of a medicament for the treatment of disorders, defects and inflammations caused by implants, and of osteolytic disorders such as osteoporosis, thrombosis, cardiac infarct and arteriosclerosis, and for the acceleration and strengthening of the integration process of the implant or of the biocompatible surface into the tissue.

13. Use of a compound according to one of Claims 1 to 5 for the coating, by means of covalent or adsorptive binding, of implants for human and animal organs.

## Revendications

1. Composé de formule 1
B-Q-X₁ 1
dans laquelle
B est une molécule bioactive, médiatrice de l'adhésion cellulaire, choisie parmi le groupe 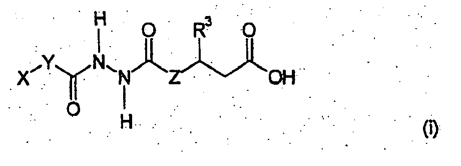 et
Thr-Trp-Tyr-Lys-Ile-Ala-Phe-Gln-Arg-Asn-Arg-Lys (ii)
Trp-Tyr-Lys-Ile-Ala-Phe-Gln-Arg-Asn-Arg-Lys (iii)
Tyr-Lys-Ile-Ala-Phe-Gln-Arg-Asn-Arg-Lys (iv)
Thr-Trp-Tyr-Lys-Ile-Ala-Phe-Gln-Arg-Asn-Arg (v)
Thr-Trp-Tyr-Lys-Ile-Ala-Phe-Gln-Arg-Asn (vi)
Thr-Trp-Tyr-Lys-Ile-Ala-Phe-Gln-Arg (vii),
où pour (i)
X représente un groupe H₂N-C(=NH)-NH, un groupe Het-NH-, un groupe H₂N-C(=NH)-, un groupe A-C(=NH)-NH- ou un groupe Het-,
Y représente un groupe -(CH₂)ₙ- ou un groupe 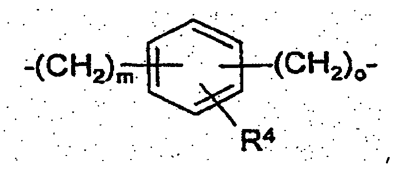 un groupe -(CH₂)ₛ-CH(R⁴)-(CH₂)ₜ- ou un groupe - (CH₂)ₚ-Het¹-(CH₂)_{q}-,
Z représente un groupe N-R² ou un groupe CH-R²
R² représente un atome d'hydrogène ou un groupe alkyle renfermant de 1 à 4 atomes de carbone,
R³ représente un atome d'hydrogène, un groupe Ar, un groupe Het ou un groupe A,
R⁴ représente un atome d'hydrogène, un groupe A, un groupe Ar, un groupe OH, un groupe OA, un groupe OAr, un groupe arylalkyle, un groupe Hal, un groupe CN, un groupe NO₂, un groupe CF₃ ou un groupe OCF₃,
A représente un groupe COOH, un groupe NH₂ ou un groupe alkyle renfermant de 1 à 6 atomes de carbone, non substitué ou substitué par un groupe COOH ou un groupe NH₂,
Ar représente un groupe phényle non substitué ou mono-, bi- ou tri-substitué par un groupe A, un groupe OH, un groupe NH₂, un groupe OA, un groupe CF₃, un groupe OCF₃, un groupe CN, un groupe NO₂ ou un groupe Hal, qui peut être substitué par un groupe phényle mono-, bi- ou tri-substitué par un groupe A, un groupe OH, un groupe OA, un groupe OCF₃, un groupe CN, un groupe NO₂ ou un groupe Hal de telle sorte qu'il se forme un groupe biphényle non substitué ou substitué,
Hal représente un atome de fluor, un atome de chlore, un atome de brome ou un atome d'iode,
Het représente un radical hétérocyclique monocyclique ou bicyclique, saturé, partiellement insaturé ou totalement insaturé, renfermant de 5 à 10 chaînons de noyau, dans lequel de 1 à 3 atomes d'azote et/ou de soufre ou d'oxygène peuvent être présents, et le radical hétérocyclique peut être mono- ou bi-substitué par un groupe CN, un groupe Hal, un groupe OH, un groupe NH₂, un groupe COOH, un groupe OA, un groupe CF₃, un groupe A, un groupe NO₂, un groupe Ar ou un groupe OCF₃,
Het¹ représente un hétérocycle aromatique à 5 ou 6 chaînons, renfermant de 1 à 4 atomes d'azote et/ou de soufre, qui peut être non substitué ou mono- ou bi-substitué par un atome de fluor, un atome de chlore, un atome de brome, un groupe A, un groupe OA ou un groupe OCF₃,
n vaut 4, 5 ou 6,
m, o, p, q valent 0, 1 ou 2,
s, t valent 0, 1, 2, 3, 4 ou 5 ;
Q est absent ou représente une molécule d'espaceur organique et
X₁ représente une molécule d'ancrage, choisie parmi le groupe
-C0-CH=CH₂ (viii)
-CO-(CH₂)₁₋₂₀-CO-CH=CH₂ (ix)
-CO-(CH₂)₁₋₂₀-SH (x)
-CO-CH(NH₂)-CH₂-SH (xi)
-NH-(CH₂)₁₋₂₀-CO-CH=CH₂ (xii)
-NH-(CH₂)₂₋₂₀-SH (xiii)
-NH-CH(CO₂H)-CH₂-SH (xiv),
où dans le cas des composés (viii) à (xi), un groupe amino libre du groupe B est lié de façon analogue à un peptide à un groupe carboxyle libre de la molécule d'espaceur Q ou de la molécule d'ancrage X₁, ou un groupe amino libre du radical Q est lié de façon analogue à un peptide à un groupe carboxyle libre du radical X₁, et dans le cas des composés (xii) à (xiv), un groupe carboxyle libre du groupe B est lié de façon analogue à un peptide à un groupe amino libre de la molécule d'espaceur Q ou de la molécule d'ancrage X₁, ou un groupe carboxyle libre du radical Q est lié de façon analogue à un peptide à un groupe amino libre du radical X₁,
ainsi que des sels de celui-ci.

2. Composé selon la revendication 1, dans lequel Q est choisi parmi le groupe
[CO-(CH₂)ₓ-NH-]ₘ (xvii)
[CO-CH₂-(O-CH₂CH₂)_{y}-NH-]ₘ (xviii)
[CO-(CH₂)_{z}-CO-] (xix)
[NH-(CH₂)_{z}-NH-] (xx)
[CO-CH₂-(OCH₂CH₂)_{y}-O-CH₂-CO-] (xxi)
[NH-CH₂CH₂-(OCH₂CH₂)_{y}-NH- (xxii)
ainsi que leur combinaison,
dans lesquels
m vaut, dans chaque cas indépendamment, de 1 à 20,
x vaut de 1 à 12,
y vaut de 1 à 50,
z vaut de 1 à 12.

3. Composé selon la revendication 1, dans lequel Q est choisi parmi le groupe
[CO-(CH₂)ₓ-NH-]ₘ (xvii)
[CO-CH₂-(O-CH₂CH₂)_{y}-NH-]ₘ (xviii)
[CO-(CH₂)_{z}-CO-] (xix)
[NH-(CH₂)_{z}-NH-] (xx)
[CO-CH₂-(OCH₂CH₂)_{y}-O-CH₂-CO-] (xxi)
[NH-CH₂CH₂-(OCH₂CH₂)_{y}-NH-] (xxii)
ainsi que leur combinaison,
dans lesquels
m vaut, dans chaque cas indépendamment, de 1 à 8,
x vaut de 1 à 5,
y vaut de 1 à 6 et
z vaut de 1 à 6.

4. Composés de formule 1 selon la revendication 1
a) 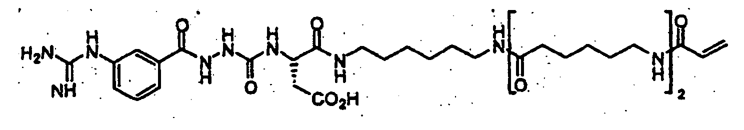
b) Thr-Trp-Tyr-Lys-Ile-Ala-Phe-Gln-Arg-Asn-Arg-Lys-Aca-Aca-Cys
c) Cys-Aca-Aca-Thr-Trp-Tyr-Lys-Ile-Ala-Phe-Gln-Arg-Asn-Arg-Lys
e) Cys-Aca-Aca-Thr-Trp-Tyr-Lys-Ile-Ala-Phe-Gln-Arg.

5. Composés de formule 1 selon la revendication 1, dans laquelle B est choisi parmi (ii), (iv) ou (v) :
d) Lys-Ile-Ala-Phe-Gln-Arg-Asn-Arg-Lys-Aca-Aca-Cys
f) Phe-Gln-Arg-Asn-Arg-Lys-Aca-Aca-Cys
g) Cys-Aca-Aca-Thr-Trp-Tyr-Lys-Ile-Ala.

6. Composé selon l'une quelconque des revendications 1 à 5, en tant que médicament destiné au traitement de maladies, de défauts et d'inflammations causés par des implants, et de maladies ostéolytiques telles que l'ostéoporose, la thrombose, l'infarctus cardiaque et l'artériosclérose, ainsi qu'à l'accélération et au renforcement du processus d'intégration de l'implant ou de la surface biocompatible dans le tissu.

7. Implant, approprié pour des organes humains et animaux, constitué essentiellement d'une matrice de support et d'une couche d'une molécule bioactive, médiatrice de l'adhésion cellulaire, enveloppant cette matrice, **caractérisé en ce que** la couche enveloppante est formée d'un composé selon les revendications 1 à 5, une liaison covalente ou adsorbante étant présente entre la matrice de support et ce composé.

8. Implant selon la revendication 7, **caractérisé en ce que** la matrice du support et/ou sa surface sont un métal ou un oxyde métallique.

9. Implant selon la revendication 7, **caractérisé en ce que** la matrice du support et/ou sa surface sont un polymère.

10. Implant selon la revendication 9, **caractérisé en ce que** le polymère est un polyméthacrylate de méthyle, un polyméthacrylate d'hydroxyéthyle ou leurs copolymères.

11. Procédé de préparation d'un composé de formule 1 selon la revendication 1 ainsi que de ses sels, **caractérisé en ce qu'**une molécule bioactive B, qui peut être pourvue de groupes protecteurs, et une molécule d'espaceur-ancrage (Q-X₁) ou une molécule d'ancrage (X₁), pourvue de groupes protecteurs, sont liées l'une à l'autre de façon peptidique, et les groupes protecteurs sont ensuite éliminés, et/ou **en ce qu'**un composé basique ou acide de formule 1 est converti en l'un de ses sels par un traitement avec un acide ou une base.

12. Utilisation d'un composé selon l'une quelconque des revendications 1 à 5 pour la production d'un médicament destiné au traitement de maladies, de défauts et d'inflammations causés par des implants, et de maladies ostéolytiques telles que l'ostéoporose, la thrombose, l'infarctus cardiaque et l'artériosclérose, ainsi qu'à l'accélération et au renforcement du processus d'intégration de l'implant ou de la surface biocompatible dans le tissu.

13. Utilisation d'un composé selon l'une quelconque des revendications 1 à 5 pour l'enrobage, au moyen d'une liaison covalente ou adsorbante, d'implants pour des organes humains et animaux.
